(19) **Europäisches Patentamt**
**European Patent Office**
**Office européen des brevets**

(11) **EP 2 478 375 B1**

(12) **EUROPEAN PATENT SPECIFICATION**

(45) Date of publication and mention
of the grant of the patent:
**15.04.2015 Bulletin 2015/16**

(51) Int Cl.:
**G01N 33/68** (2006.01)

(21) Application number: **10755214.3**

(86) International application number:
**PCT/GB2010/001723**

(22) Date of filing: **13.09.2010**

(87) International publication number:
**WO 2011/033252 (24.03.2011 Gazette 2011/12)**

(54) **DIAGNOSTIC AGENT FOR PARKINSON'S DISEASE**

DIAGNOSTIKUM FÜR MORBUS PARKINSON

AGENT DE DIAGNOSTIC POUR LA MALADIE DE PARKINSON

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **16.09.2009 GB 0916264
30.06.2010 GB 201011045**

(43) Date of publication of application:
**25.07.2012 Bulletin 2012/30**

(73) Proprietor: **United Arab Emirates University
Abu Dhabi (AE)**

(72) Inventor: **EL-AGNAF, Omar
Al Ain (AE)**

(74) Representative: **Sexton, Jane Helen
J A Kemp
14 South Square
Gray's Inn
London WC1R 5JJ (GB)**

(56) References cited:
**WO-A2-03/069332    US-A1- 2005 202 508**

- EL-AGNAF OMAR M A ET AL: "Detection of oligomeric forms of alpha-synuclein protein in human plasma as a potential biomarker for Parkinson's disease" FASEB JOURNAL,, vol. 20, no. 3, 1 March 2006 (2006-03-01), pages 419-425, XP009109458
- EL-AGNAF OMAR M A ET AL: "Review: Formation and properties of amyloid-like fibrils derived from alpha-synuclein and related proteins" JOURNAL OF STRUCTURAL BIOLOGY, vol. 130, no. 2-3, June 2000 (2000-06), pages 300-309, XP002606923 ISSN: 1047-8477
- NOGUCHI-SHINOHARA M ET AL: "CSF alpha-synuclein levels in dementia with Lewy bodies and Alzheimer's disease" BRAIN RESEARCH, ELSEVIER, AMSTERDAM, NL LNKD- DOI: 10.1016/J.BRAINRES.2008.11.055, vol. 1251, 28 January 2009 (2009-01-28), pages 1-6, XP025771524 ISSN: 0006-8993 [retrieved on 2008-11-28]

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the Invention**

The present invention relates to method of identifying whether or not an individual has Parkinson's disease (PD). In particular, the invention relates to a method for identifying whether or not an individual has PD in the pre-symptomatic phase of the disease.

**Background of the Invention**

Parkinson's disease (PD) is the second most common neurodegenerative disorder after Alzheimer's disease. The earliest clinical features of PD are typically identified retrospectively and are not specific for PD. These symptoms are typically non-motor symptoms such as constipation, depression, hyposmia, and sleep disorders.

The more generally recognised symptoms of PD are motor symptoms such as bradykinesia, muscle tremor, rigidity and balance problems. These symptoms do not appear until much later in the disease, when major damage to the brain has already occurred. Indeed, at least 70% of nigral neurons in the midbrain must be lost prior to the appearance of symptoms of this type. Even then, the first manifestation of motor symptoms may be subtle and can go unnoticed for months or years.

Accurate clinical diagnosis of PD during life is highly difficult, being correct in only about 85% of cases. As a consequence, the discovery of effective disease-modifying therapies has been impeded. The most advanced methods presently available for PD diagnosis are based on imaging using radioactive nuclides. These methods are costly and it remains a matter of debate how closely they reflect the underlying pathologic process.

To date, there is no test which can reliably determine whether or not an individual has PD. In particular, there is no test which can determine whether or not an individual has PD during the early (pre-symptomatic) phase of the disease, before too much damage to the brain has occurred.

El-Agnaf et al, FASEB Journal (2006) 20(3) p 419-425 discloses detection of soluble oligomeric forms of α-synuclein in human plasma and post-mortem cerebrospinal fluid samples.

However, there is a long-felt need for the development of a reliable test for PD which can be used on living subjects. There is a particular need for a test which can determine whether or not an individual has PD during the early (pre-symptomatic) phase of the disease.

**Summary of the Invention**

The discovery of mutations in the gene encoding α-synuclein (SNCA) in familial cases of PD first suggested a critical role for α-synuclein (α-syn) protein in the etiology of idiopathic PD. More recently, genetic studies have also revealed that rare triplication events in SNCA can be associated with severe forms of young-onset, familial PD that also feature dementia with Lewy body-type changes. Furthermore, duplication mutations cause a familial PD phenotype that more closely resembles late-onset, idiopathic PD. These collective studies indicate that an increased expression of wild-type α-syn enhances the risk of developing PD and that the protein level of α-syn might be an important determinant of the progression and severity of the Parkinsonian phenotype.

Population data also demonstrates that common genetic variability in the SNCA locus is a risk factor in sporadic PD. In addition, the neuropathologic lesions (Lewy bodies (LBs)) that best characterize end-stage PD are largely comprised of deposits of fibrillar α-syn. However, α-syn is mainly expressed by neuronal cells, and was generally considered to exist as a cytoplasmic and lipid vesicle-associated protein, which tended to discourage its use as a diagnostic marker.

It was recently shown that α-syn is in fact normally released by neuronal cells, and is present in human cerebrospinal fluid (CSF) and peripheral plasma, although levels of α-syn in CSF taken from patients with PD were found to be decreased compared to those of age-matched controls.

There is evidence suggesting that soluble oligomers of the amyloidogenic proteins associated with neurodegenerative diseases are the pathogenic species that cause neuronal cell death, rather than the mature amyloid fibrils. It has been recently shown that soluble α-syn oligomers are elevated in the brain homogenates of deceased patients with PD and dementia with Lewy bodis (DLB), relative to homogenates from normal brains. In addition, in contrast to the result in CSF, the levels of soluble α-syn oligomers in plasma have recently been found to be elevated in patients with PD compared with controls. It should be noted that the levels of α-syn oligomers in plasma obtained from PD patients varied widely and overlapped considerably with those in samples from controls.

Nonetheless, the Inventor has compared the levels of soluble α-syn oligomers in CSF taken from living PD patients and age-matched subjects including patients with Progressive supranuclear palsy (PSP) and Alzheimer's Disease (AD), as well as normal control subjects. Total levels of α-syn were also measured in order to measure overall protein levels of α-syn in the same samples. The Inventor has found that the amount of soluble α-syn oligomers and the ratio between the amount of said oligomers and the total amount of α-syn were significantly increased in CSF samples from living patients with PD, compared with CSF samples from age-matched normal controls. The Inventor has also found that the amount of soluble α-syn oligomers is significantly increased in CSF samples from patients with PD, compared with CSF samples from age-matched patients with other neurological disorders.

Accordingly, the present invention provides:

A method of identifying whether or not an individual has Parkinson's disease (PD) and/or distinguishing between PD and another neurological disorder which is not PD, which method comprises measuring the amount of soluble α-synuclein oligomers and the total amount of α-synuclein in a cerebrospinal fluid (CSF) sample taken from the individual, calculating the ratio of:

$$\underline{\text{amount of α-synuclein oligomers}}$$
$$\text{total amount of α-synuclein}$$

and thereby determining whether or not the individual has PD.

In the method, determining whether or not the individual has PD may comprise determining whether or not the ratio of the amount of α-syn oligomers to the total amount of α-syn is increased relative to the ratio in sample taken from a normal individual. For example, the ratio may be increased by at least 3 fold relative to the ratio in a sample taken from a non-PD individual.

Alternatively, determining whether or not the individual has PD may comprise determining whether or not the ratio, when expressed as a percentage, is at least 6%, preferably at least 7%.

In a method of the invention, the individual subject may be suspected of being at risk of developing PD or of another neurological disorder such as PSP or AD. The individual may have a familial history of PD. The individual may or, more typically, may not have (or have been diagnosed) with any of the clinical symptoms associated with a diagnosis of PD or of another neurological disorder such as PSP or AD. The individual may or may not have (or have been diagnosed) with any one of the non-motor symptoms of PD. The individual may typically be categorised as Hoehn-Yahr grade 2 or lower.

Also disclosed is a method for detecting α-syn oligomers in a sample of CSF comprising:

-    incubating the CSF sample with a first α-syn binding reagent which is immobilised on a solid phase;
-    detecting bound α-synuclein using a second α-syn binding reagent which is not immobilised and which binds to the same or an overlapping site on α-synuclein as the first agent.

Also disclosed is a method for detecting α-syn oligomers in a sample of CSF comprising:

-    incubating the CSF sample with a first reagent that is specific to amyloid oligomers but does not bind specifically to α-synuclein, and which is immobilised on a solid phase;
-    detecting bound amyloid oligomers using a α-synuclein binding reagent which is not immobilised and which binds specifically to α-synuclein protein.

In this embodiment the first reagent typically specifically binds a component of amyloid oligomers which is not α-synuclein. For example, the first reagent may specifically bind β-amyloid protein, Huntington protein or amylin protein.

In the method, the α-syn binding reagents may be an α-syn-specific monoclonal antibody, optionally a labelled antibody.

Also disclosed is a method of delaying or preventing the onset of PD symptoms in an individual, comprising;

(i) determining whether or not an individual has PD using a method according to the invention; and

(ii) administering to an individual identified as having PD in (i), a therapeutically effective amount of an agent that directly or indirectly inhibits α-syn aggregation and/or toxicity, an agent that reduces expression of the α-syn protein, an agent that directly or indirectly enhances or stimulates the degradation of α-syn aggregates, or a neuroprotective agent.

In the method, the neuroprotective agent may be an anti-apoptotic, an anti-oxidant, an anti-glutamatergic, a monoamine oxidase B inhibitor, an adenosine antagonist, a dopamine agonist, a mitochondrial stabiliser or a trophic factor.

The neuroprotective agent may be rasagiline, selegiline, ropinirole, pramipexole, nicotine, minocycline, creatine, caffeine, or coenzyme Q10.

Also disclosed is a test kit for use in a method for determining whether or not an individual has PD, which test kit comprises means for the detection of soluble $\alpha$-syn oligomers in a sample of CSF.

The test kit may additionally comprise means for the measurement of the total amount of $\alpha$-syn in a sample of CSF.

The methods or test kit disclosed herein can be used in clinical trials to measure the effect of drugs in both PD animal models and human PD patients.

## Summary of Figures

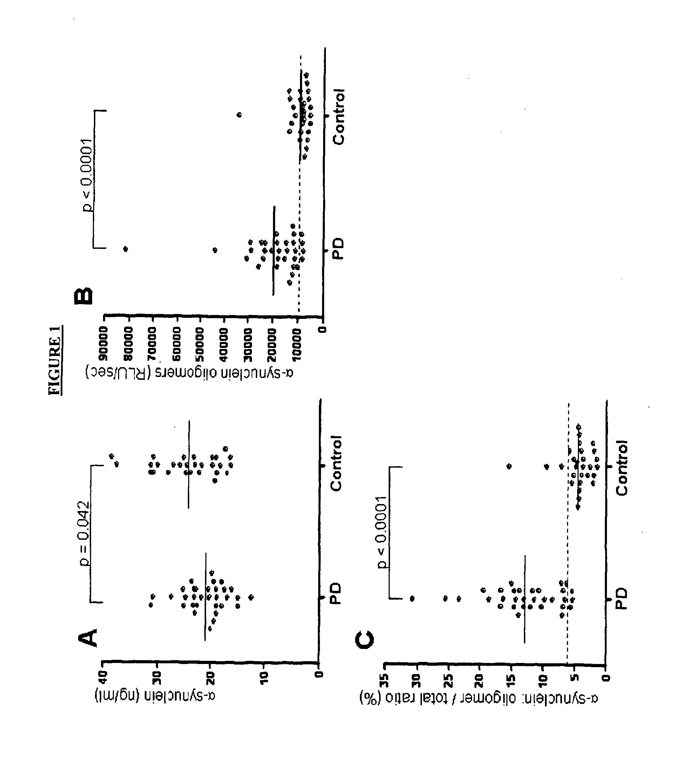

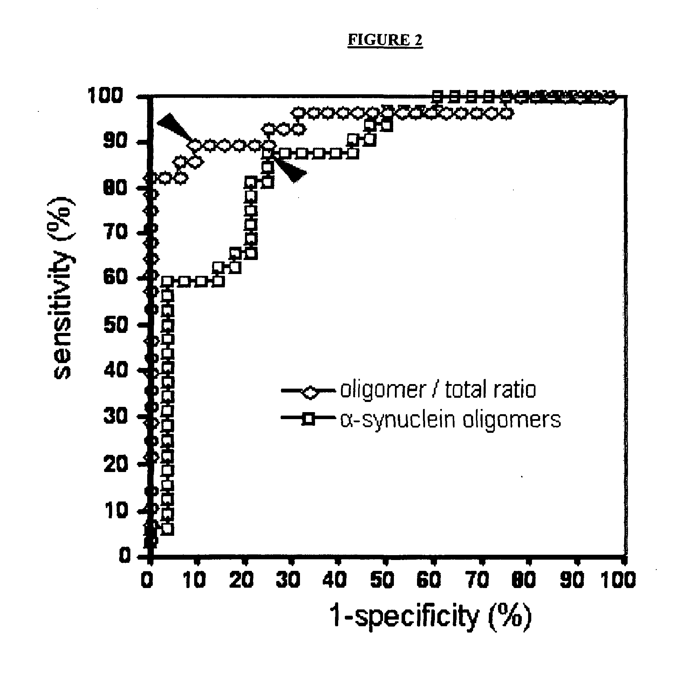

Figure 1 shows individual values for the level of total $\alpha$-synuclein (A), $\alpha$-syn oligomers (B; RLU = relative luminescence units) and the ratio of $\alpha$-syn oligomers to total $\alpha$-syn (C; oligomer/total ratio, %) in CSF from PD patients (solid circles) and controls (open circles). Each bar represents the mean value. Dashed lines in (B) and (C) indicate respective cut-off values that yield the most reliable sensitivity and specificity identified by receiver operating characteristic curves (see Figure 2).

Figure 2. Receiver operating characteristic (ROC) curves for the levels of CSF $\alpha$-syn oligomers (open square) and the ratio of $\alpha$-syn oligomers to total $\alpha$-syn in CSF (open circle) in discrimination of PD from controls over a range of cut-off points, an arrowhead indicates a cut-off value that yields the most appropriate sensitivity and specificity

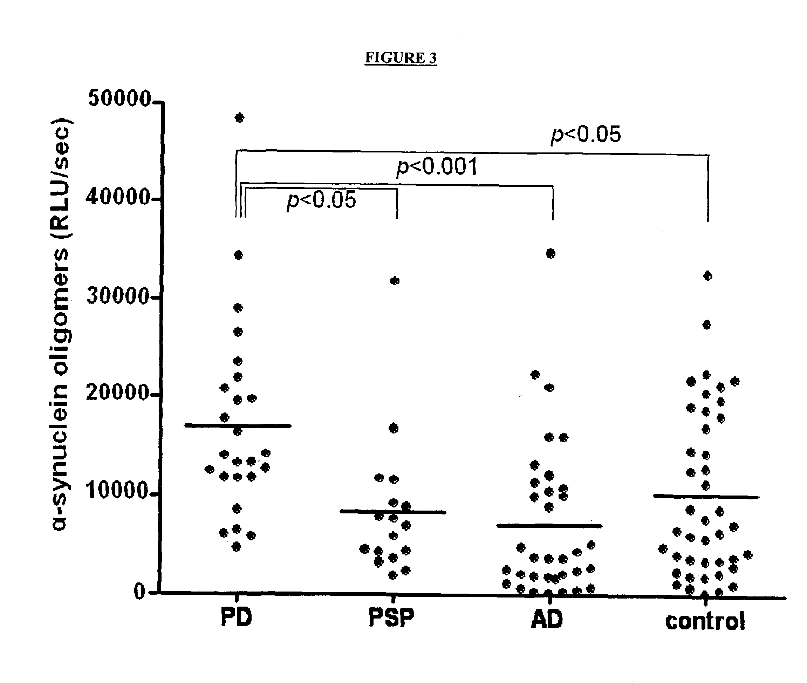

Figure 3 shows individual values of the level of $\alpha$-syn oligomers in CSF from a second cohort of subjects including patients with PD (n = 25), PSP (Progressive supranuclear palsy; n = 18), AD (Alzheimer's Disease; n = 35) and normal control subjects (n = 43). Each bar represents the mean value.

## Detailed description of the Invention

The present invention relates to a method of identifying whether or not a subject has PD. The invention therefore relates to the detection of PD in the individual subject. The individual is typically a mammal. The mammal is typically a human or a domestic mammal such as a horse, a cow, a sheep, a dog or a cat. The individual is preferably a human. The individual may be up to 30, up to 40, up to 50, up to 60 or up to 70 years old. The individual may have an age of 30 to 40, 30 to 50, 30 to 60 or 30 to 70 years. The individual may have an age of 40 to 50, 40 to 60 or 40 to 70 years. The individual may have an age of 50 to 60 or 50 to 70 years.

The individual may display no clinical symptoms associated with PD, or the individual may exhibit one or more of the following symptoms:

Primary Motor Symptoms

Present clinical practice typically requires the presence of at least one primary motor symptom for a diagnosis of PD. The primary motor symptoms are:

(i) Resting Tremor: About 70 percent of people with Parkinson's experience a slight tremor, which is often the first identifiable symptom. The tremor is typically in either the hand or foot on one side of the body, or less commonly in the jaw or face. The tremor appears as a "beating" or oscillating movement. The Parkinson's tremor usually appears when a person's muscles are relaxed, hence it is called "resting tremor." Typically the affected body part trembles when it is not doing work, and the tremor subsides when a person begins an action. The tremor often spreads to the other side of the body as the disease progresses, but remains most apparent on the original side of occurrence.

(ii) Bradykinesia (Slow movement): the patient displays markedly slow movement. In addition to slow movement, a person with bradykinesia will typically also have incomplete movement, difficulty initiating movements and difficulty in suddenly stopping ongoing movements. People who have bradykinesia may walk with short, shuffling steps (festination). Bradykinesia and rigidity can occur in the facial muscles, reducing a person's range of facial expressions and resulting in a "mask-like" appearance.

(iii) Rigidity: also called increased muscle tone, means stiffness or inflexibility of the muscles. In rigidity, the muscle tone of an affected limb is always stiff and does not relax, sometimes resulting in a decreased range of motion. For example, a person who has rigidity may not be able to swing his or her arms when walking because the muscles are too tight. Rigidity can cause pain and cramping.

(iv) Postural Instability (Impaired Balance and Coordination): Subjects with PD often experience instability when standing, or have impaired balance and coordination. These symptoms, combined with other symptoms such as bradykinesia, increase the chance of falling. Subjects with balance problems may have difficulty making turns or abrupt movements. The subject may go through periods of "freezing," in which the subject finds it difficult to start walking. Slowness and incompleteness of movement can also affect speaking and swallowing.

Secondary Motor Symptoms

Not all PD subjects will experience secondary motor symptoms. However, most subjects typically exhibit one or more of the following:

- Stooped posture, a tendency to lean forward
- Dystonia
- Fatigue
- Impaired fine motor dexterity and motor coordination
- Impaired gross motor coordination
- Poverty of movement (decreased arm swing)
- Akathisia
- Speech problems, such as softness of voice or slurred speech caused by lack of muscle control
- Loss of facial expression, or "masking"
- Micrographia (small, cramped handwriting)
- Difficulty swallowing
- Sexual dysfunction
- Drooling

Non-motor Symptoms

A number of non-motor symptoms are associated with PD. However, these symptoms are not specific for PD, and are typically only identified as indicating PD retrospectively. That is, the non-motor symptoms experienced by a subject are not typically recognised as indicating PD until after the presence of primary and secondary motor symptoms has been confirmed. Even so, a PD patient will typically exhibit one or more of the following:

- Pain
- Dementia or confusion
- Sleep disturbances (e.g. REM sleep behaviour disorder (RBD))
- Hyposmia
- Constipation
- Skin problems
- Depression
- Fear or anxiety
- Memory difficulties and slowed thinking
- Urinary problems
- Fatigue and aching
- Loss of energy
- Compulsive behavior (e.g. Gambling)
- Cramping

The individual to be tested typically does not have or has not been diagnosed with any of the primary motor symptoms of PD. Preferably, the individual also does not have or has not been diagnosed with any of the secondary motor symptoms of PD

The individual may be suspected of being at risk of developing PD because of the presence of one or more of the non-motor symptoms or secondary motor symptoms of PD.

The individual may or may not have been categorised according to the Hoehn-Yahr scale. The Hoehn-Yahr scale is a commonly used system for describing how the symptoms of Parkinson's disease progress. The scale allocates stages from 0 to 5 to indicate the relative level of disability:

- Stage one: Symptoms on one side of the body only.
- Stage two: Symptoms on both sides of the body. No impairment of balance.
- Stage three: Balance impairment. Mild to moderate disease. Physically independent.
- Stage four: Severe disability, but still able to walk or stand unassisted.
- Stage five: Wheelchair-bound or bedridden unless assisted.

If categorised, the individual is typically grade 2 or lower on the Hoehn-Yahr scale.

The individual may or may not have been diagnosed with PD according to the UK Parkinson's Disease Society Brain Bank criteria. These criteria are:

*Step 1: Diagnosis of Parkinsonian Syndrome*

- Bradykinesia
- At least one of the following

    - Muscular rigidity
    - 4-6 Hz rest tremor
    - postural instability not caused by primary visual, vestibular, cerebellar, or proprioceptive dysfunction

*Step 2: Identification of features tending to exclude Parkinson's disease as the cause of Parkinsonism*

- history of repeated strokes with stepwise progression of parkinsonian features
- history of repeated head injury
- history of definite encephalitis
- oculogyric crises
- neuroleptic treatment at onset of symptoms
- more than one affected relative
- sustained remission
- strictly unilateral features after 3 years
- supranuclear gaze palsy
- cerebellar signs
- early severe autonomic involvement
- early severe dementia with disturbances of memory, language, and praxis
- Babinski sign
- presence of cerebral tumor or communication hydrocephalus on imaging study
- negative response to large doses of levodopa in absence of malabsorption
- MPTP (1-methyl 4-phenyl 1, 2, 3, 6-tetrahydropyridine) exposure

*Step 3: Identification of features that support a diagnosis of Parkinson 's disease (three or more in combination with step 1 required for diagnosis of definite Parkinson 's disease):*

- Unilateral onset
- Rest tremor present
- Progressive disorder
- Persistent asymmetry affecting side of onset most
- Excellent response (70-100%) to levodopa
- Severe levodopa-induced chorea
- Levodopa response for 5 years or more
- Clinical course of ten years or more

The individual may or may not be suspected of having or being at risk of developing another neurological disorder such as PSP or AD. The individual may or may not have been diagnosed with such a disorder, or with one or more symptoms associated with such a disorder.

The individual may be suspected of being at risk of developing PD because of the presence of one or more factors which increase susceptibility to PD.

For example, the individual may have a familial history of PD. Large epidemiological studies demonstrate that people with an affected first-degree relative, such as a parent or sibling, have a two-to-three fold increased risk of developing Parkinson's, as compared to the general population.

The individual may have a mutation or polymorphism in a gene or locus associated with PD. For example, the individual may have a mutation or polymorphism in one of more of the following genes or loci: PARK1 (gene encoding $\alpha$-synuclein (SNCA)), PARK2 (gene encoding suspected ubiquitin-protein ligase Parkin (PRKN2)), PARK3, PARK4, PARK5 (gene encoding ubiquitin carboxy-terminal hydrolase L1), PARK6 (gene encoding a putative protein kinase (PINK1)), PARK7 (gene encoding DJ-1), or PARK8 (gene encoding leucine-rich repeat kinase 2 (LRRK2)).

The individual may have a mutation or polymorphism in one or more of the genes encoding the following products: Dopamine receptor 2, Dopamine receptor 4, Dopamine transporter, Monoamine oxidase A, Monoamine oxidase B, Catechol-o-methyl-transferase, N-acetyl transferase 2 detoxification enzyme, Apo-lipoprotein E, Glutathione transferase detoxification enzyme T1, Glutathione transferase detoxification enzyme MI, Glutathione transferase detoxification enzyme, or Glutathione transferase detoxification enzyme Z1; and/or in the tRNA Glu mitochondrial gene and/or the Complex 1 mitochondrial gene. Preferably the individual has a mutation or polymorphism in the gene encoding Monoamine oxidase B, and/or N-acetyl transferase 2 detoxification enzyme, and/or Glutathione transferase detoxification enzyme T1 and/or in the tRNA Glu mitochondrial gene.

Environmental risk factors may also be present. To date, epidemiological research has identified rural living, well water, herbicide use and exposure to pesticides as factors that may be linked to PD. Also, MPTP (1-methyl 4-phenyl 1, 2, 3, 6-tetrahydropyridine) can cause Parkinsonism if injected. The chemical structure of MPTP is similar to the widely used herbicide paraquat and damages cells in a way similar to the pesticide rotenone, as well as some other substances.

The present invention involves measuring the amount of soluble $\alpha$-syn oligomers in a cerebrospinal fluid (CSF) sample taken from an individual. An increased level of soluble $\alpha$-syn oligomers compared with the baseline level may indicate that the individual has PD. The baseline level is typically the amount of soluble $\alpha$-syn oligomers in a CSF sample from a non-PD individual. The non-PD individual is typically an age-matched neurologically normal individual: referred to herein as a "normal individual". Alternatively the non-PD individual may be an age-matched patient who has been diagnosed with one or more symptoms associated with another neurological disorder which is not PD. For example, the non-PD individual may have been diagnosed with one or more symptoms of PSP or AD.

The inventors have shown that the amount or level of soluble $\alpha$-syn oligomers in a sample from a PD patient is typically at least 1.5 fold, preferably at least 2 fold, more preferably at least 3 fold higher than the amount or level in a sample from a non-PD individual.

However, the present invention also involves measurement of the total amount of $\alpha$-syn in a sample, and assessment of the ratio of the amount of soluble $\alpha$-syn oligomers to the total amount $\alpha$-syn in the sample.

According to the present invention, an increased oligomers/total $\alpha$-syn ratio indicates that the individual has PD. For example, the Inventors have shown that when the oligomers/total $\alpha$-syn ratio for a given sample is expressed as a percentage, a level of at least 5%, preferably at least 6%, more preferably at least 7%, indicates that the individual has PD.

Also according to the present invention, an increased oligomers/total $\alpha$-syn ratio compared with the baseline ratio indicates that the individual has PD. The baseline ratio is typically the oligomers/total $\alpha$-syn ratio in a sample from a non-PD individual. The non-PD individual is typically an age-matched neurologically normal individual: referred to herein as a "normal individual". Alternatively the non-PD individual may be an age-matched patient who has been diagnosed with one or more symptoms associated with another neurological disorder which is not PD. For example, the non-PD individual may have been diagnosed with one or more symptoms of PSP or AD. The increase in oligomers/total $\alpha$-syn ratio associated with PD is typically at least 1.5 fold, 2 fold, 2.5 fold or 3 fold relative to the baseline ratio.

The invention is typically carried out *in vitro* on a cerebrospinal fluid sample obtained from the individual. The sample may be typically processed prior to being assayed, for example by centrifugation. The sample may also be stored prior to assay, preferably below -70°C.

Standard methods known in the art may be used to assay the level of soluble $\alpha$-syn oligomers. These methods typically involve using an agent for the detection of soluble $\alpha$-syn oligomers. The agent typically binds specifically to soluble $\alpha$-syn oligomers. The agent may be an antibody specific for soluble $\alpha$-syn oligomers. By specific, it will be understood that the agent or antibody binds to soluble $\alpha$-syn oligomers with no significant cross-reactivity to any other molecule, particularly any other protein. For example, an agent or antibody specific for soluble $\alpha$-syn oligomers will show no significant cross-reactivity with monomeric $\alpha$-syn. Cross-reactivity may be assessed by any suitable method.

Alternatively, the Inventor has developed a novel method for detection of soluble $\alpha$-syn oligomers, particularly in CSF samples. The method is based on a sandwich ELISA technique. ELISA is a heterogeneous, solid phase assay that requires the separation of reagents. The sandwich ELISA technique requires two agents: a capture agent and a detection agent. The first agent specifically binds the target and is bound to a solid support (is immobilized). The second agent is attached to a marker, typically an enzyme conjugate. A substrate for the enzyme is used to quantify the target-agent complex and hence the amount of target in a sample. The solid supports for ELISA reactions preferably comprise wells. The agents are typically antibodies.

The assay developed by the inventor typically uses as a capture agent a specific anti-$\alpha$-syn monoclonal antibody. However, unlike conventional ELISA, the detection agent in the assay of the invention binds to the same or an overlapping site on α-syn as the capture agent. For example, where the agents are antibodies, the detection agent is typically an antibody that recognizes the same epitope as the capture agent. Monomeric α-syn cannot give a signal in the assay of the invention because the capture agent occupies the only binding site available on the protein. However, in the case of oligomeric forms of α-syn, multiple binding sites are available, permitting binding by both the capture agent and the detection agent. The capture agent and the detection agent may have identical antigen recognition sites. In a preferred embodiment, the detection agent is a biotinylated antibody. Detection is then achieved via avidin-conjugated horse radish peroxidase (HRP) enzyme, monitored by incubation with an appropriate detectable substrate, preferably a chemiluminnescent substrate. That is, the "read-out" of the assay is a luminescence level, typically shown as Relative Luminescence Units.

Total α-syn levels are measured using any conventional method. These methods typically involve using an agent for the detection of all forms of α-syn. The agent typically binds specifically to all forms of α-syn oligomers. The agent may be an antibody specific for α-syn. A preferred method is a conventional sandwich ELISA assay. That is, wherein the capture and detection antibodies are different. Ideally, the form of the "read out" for the method for measuring total α-syn levels should be the same as that for the method for measuring soluble α-syn oligomer levels. This facilitates calculation of the oligomers / total α-syn ratio.

Also disclosed is an alternative method for detecting α-syn oligomers in a sample of CSF, which takes advantage of the presence of different protein components that may be present in said oligomers. Typically an α-syn oligomer may also comprise, for example, β-amyloid protein, Huntington protein or amylin protein. An oligomer comprising multiple different proteins may typically be referred to by the general term "amyloid oligomer". Thus, the method of the invention may incubate the CSF sample with a first reagent that is specific to amyloid oligomers but does not bind specifically to α-synuclein, and which is immobilised on a solid phase. Detection of the oligomers is then achieved using a α-synuclein binding reagent which is not immobilised and which binds specifically to α-synuclein protein, as in the methods described above..

An antibody used in any method disclosed herein may either be a whole antibody or a fragment thereof which is capable of binding to the desired protein, for example the desired form of α-syn. The antibody may be monoclonal. Such a whole antibody is typically an antibody which is produced by any suitable method known in the art. For example, polyclonal antibodies may be obtained by immunising a mammal, typically a rabbit or a mouse, with α-syn under suitable conditions and isolating antibody molecules from, for example, the serum of said mammal. Monoclonal antibodies may be obtained by hybridoma or recombinant methods.

Typically the antibody is a mammalian antibody, such as a primate, human, rodent (e.g. mouse or rat), rabbit, ovine, porcine, equine or camel antibody. The antibody may be a camelid antibody or shark antibody. The antibody may be a nanobody. The antibody can be any class or isotype of antibody, for example IgM, but is preferably IgG. The fragment of whole antibody that can be used in the method comprises an antigen binding site, e.g. Fab or F(ab)2 fragments. In one embodiment the antibody is a chimeric antibody comprising sequence from different natural antibodies, for example a humanised antibody.

Also disclosed is a diagnostic kit that comprises means for measuring the level of soluble α-syn oligomers in a sample, and thereby determining whether or not the individual has PD. The kit typically contains one or more antibodies that specifically bind α-syn. For example, the kit may comprise a monoclonal antibody, a polyclonal antibody, a single chain antibody, a chimeric antibody, a CDR-grafted antibody or a humanized antibody. The antibody may be an intact immunoglobulin molecule or a fragment thereof such as a Fab, F(ab')$_2$ or Fv fragment. If more than one antibody is present, the antibodies preferably have overlapping determinants such that they may be used to detect soluble α-syn oligomers but not monomers, in accordance with the assay developed by the inventor.

The kit may additionally comprise means for the measurement of the total α-syn in a sample.

The kit may additionally comprise one or more other reagents or instruments which enable any of the embodiments of the method mentioned above to be carried out. Such reagents or instruments include one or more of the following: suitable buffer(s) (aqueous solutions), means to isolate α-syn from a sample, means to obtain a sample from the individual (such as a vessel or an instrument comprising a needle) or a support comprising wells on which quantitative reactions can be done. The kit may, optionally, comprise instructions to enable the kit to be used in the method of the invention or details regarding which individuals the method may be carried out upon.

Also disclosed is a method of delaying or preventing the onset of PD symptoms in an individual. The method comprises: (i) determining whether or not an individual has PD using a method according to the invention; and (ii) administering to an individual identified as having PD in (i), a therapeutically effective amount of an agent that directly or indirectly inhibits α-syn aggregation and/or toxicity, an agent that reduces expression of the α-syn protein, an agent that direcectly or indirectly enhances or stimulates the degradation of α-syn aggregates, or a neuroprotective agent. The neuroprotective agent is typically an anti-apoptotic, an anti-oxidant, an anti-glutamatergic, a monoamine oxidase B inhibitor, an adenosine antagonist, a dopamine agonist, a mitochondrial stabiliser or a trophic factor. For example, the neuroprotective agent may be rasagiline, selegiline, ropinirole, pramipexole, nicotine, minocycline, creatine, caffeine, or coenzyme Q10.

The following Example illustrates the invention:

## Example 1

Subjects and Methods

*Subjects*

This study complied with the Declaration of Helsinki and was approved by the University Ethics Committee (Kyoto Prefectural University of Medicine, Kyoto, Japan). All of the subjects provided their written informed consent to participate in the study. 32 patients with clinically defined PD (18 men and 14 women, aged 43-83 [mean $\pm$ SD, 67.3 $\pm$ 9.4] years, see Table 1 for clinical details) were enrolled in this study, who were diagnosed according to the UK Parkinson's Disease Society Brain Bank criteria.

Three out of the 32 PD patients had dementia that had developed 1 year or more after the onset of motor symptoms, and were thereby diagnosed as PD with dementia, not as dementia with Lewy bodies. The age-matched control subjects (18 men and 10 women, aged 29-86 [mean $\pm$ SD, 64.0 $\pm$ 13.9] years) consisted of neurologically normal individuals who underwent lumbar puncture as part of diagnostic process (n = 15) and controls with various neurological disorders (n = 13) including patients with lacunar infarction in pons (n = 1), epilepsy (n = 2), myelopathy (n = 1), peripheral neuropathy (n = 6), and myopathy (n = 2). None of the 28 age-matched control patients had dementia. Fresh CSF samples were collected from the patients with PD and the control subjects, divided into aliquots, and then stored at -80°C until used for immunoassays with our ELISA systems.

*Immunoassay for Total $\alpha$-Synuclein in CSF*

Total $\alpha$-syn in the CSF samples was measured using a sandwich ELISA assay with some modification to improve the sensitivity of the assay to measure $\alpha$-syn directly from the CSF samples. An anti-human $\alpha$-syn monoclonal antibody 211 (Santa Cruz Biotechnology, USA) was used for capturing, and anti-human $\alpha$-syn polyclonal antibody FL-140 (Santa Cruz Biotechnology, USA) was used for antigen detection through a horseradish peroxidase (HRP)-linked chemiluminescence assay. ELISA plate (Nunc Maxisorb, NUNC, Denmark) was coated for overnight incubation at 4°C, with 1 $\mu$g/ml of 211 (100 $\mu$l/well), in 200 mM NaHCO3, pH 9.6. After incubation for 2 hours with 200 $\mu$l/well of blocking buffer (phosphate-buffered saline (PBS) containing 2.5% gelatin and 0.05% Tween 20), 100$\mu$l of the CSF samples were then added to each well and incubated at 37 °C for 3 hrs. After the incubation, captured $\alpha$-syn protein was detected by the reaction with 100 $\mu$l/well of FL-140 antibody (0.2 $\mu$g/ml), followed by incubation with 100 $\mu$l/well (1:10,000-dilution) of horseradish peroxidase (HRP)-labeled anti-rabbit antibody (DAKO, Denmark). Bound HRP activities were assayed by (100 $\mu$l/well) chemiluminescent reaction using an enhanced chemiluminescent substrate (SuperSignal ELISA Femto Maximum Sensitivity Substrate, Pierce Biotechnology, Rockford, USA). The chemiluminescence in relative light units was measured at 395 nm with a microplate luminometer (SpectraMax L, Molecular Device, Tokyo). The standard curve for the ELISA assay was carried out using 100 $\mu$l/well of recombinant human $\alpha$-syn solution at different concentrations of the protein in PBS. All samples and standards were run in triplicate on the same day with the same lot of standards. The relative concentration estimates of total $\alpha$-syn in CSF were calculated according to each standard curve. The intra-assay and inter-assay precision was <9%.

*Preparation of the biotinylated antibody*

Sulfo-NHS-LC-Biotin (Pierce Biotechnology, Rockford, USA) (200 $\mu$g) was reacted with the antibody to be biotinylated (1 ml at 200 $\mu$g/ml) in PBS and then placed on ice for 2 hrs. The mixture was desalted on Bio-Spin-6 columns (BIO-RAD, UK) to remove excess uncoupled biotin. The biotinylated antibodies were stored at 4°C until used.

*An ELISA to measure $\alpha$-syn oligomers*

An ELISA 384-well plate was coated via overnight incubation at 4oC with 1 $\mu$g/ml of non-biotinylated 211 mouse monoclonal antibody (MAb) (Santa Cruz Biotechnology, California, USA) in 200 mM NaHCO3 (Sigma-Aldrich Company Ltd., Dorset, U.K.) at pH 9.6 (50 $\mu$l/well). The plate was washed 4 times with PBST (PBS containing 0.05% Tween 20), and incubated with 100 $\mu$l/well of blocking buffer (PBS containing 2.5% gelatin and 0.05% Tween 20) for 2 hrs at 37 °C. The plate was then washed 4 times with PBST; 50 $\mu$l of CSF samples to be tested were added to each well, and the plate was incubated at 37°C for another 3 hrs. After washing 4 times with PBST, 50 $\mu$l of biotinylated 211 diluted to 1 $\mu$g/ml in blocking buffer was added, and incubated at 37°C for 2 hrs. The wells were washed 4 times with PBST and incubated with 50 $\mu$l/well of ExtrAvidin-Peroxidase (Sigma-Aldrich Company Ltd., Dorset, U.K.) diluted (1:10000) in blocking buffer and incubated for 1 hr at 37°C. After washing the plates were finally incubated with 50 µl/well of an enhanced chemiluminescent substrate (SuperSignal ELISA Femto Maximum Sensitivity Substrate, Pierce Biotechnology), after which chemiluminescence in relative light units was immediately measured with a Victor2 1420 (Wallac) microplate reader.

For all ELISA assays, samples were continuously kept on ice, and the assays were performed on sample aliquots after a single thaw after initial freezing.

*Statistical Analysis*

Regarding differences between the PD and control groups, the groups were compared using Mann-Whitney U test. The level of significance was set at $P<0.05$. Correlational analysis was conducted by Pearson's simple correlation. Receiver operating characteristics (ROC) were analyzed to assess the most appropriate cutoff values for the level of CSF $\alpha$-syn oligomers and the oligomer/total $\alpha$-syn ratio in CSF in the distinction between the PD and control groups. All analyses were carried out using GraphPad Prism software (GraphPad Prism Version 4.0, GraphPad software, San Diego, USA).

<u>Results</u>

In the present study 32 PD patients and 28 control subjects were investigated for total $\alpha$-syn levels. No significant difference in age or gender ratio was observed between the two groups. As shown in Figure 1A, comparison of the concentrations of total $\alpha$-syn in CSF shows considerable overlap in individual signals between the PD and control groups. Nevertheless, the results demonstrated lower mean concentrations of CSF total $\alpha$-syn in the PD patients (mean $\pm$ SEM = 20.98 $\pm$ 0.76 ng/ml, n = 32) when compared with the controls (24.40 $\pm$ 1.17, n = 28) (p = 0.0417, Mann-Whitney U test).

Next, the levels of CSF $\alpha$-syn oligomers were measured in the same aliquots of CSF derived from the same subjects. For this measurement, an ELISA system which can measure only soluble $\alpha$-syn oligomers without detecting the monomeric forms of $\alpha$-syn was used. A scatter plot of CSF $\alpha$-syn oligomers level for each PD patient and control groups is shown in Figure 1B, in which the levels of $\alpha$-syn oligomers are shown in the intensity of chemiluminescence signal (relative luminescence units / second (RLU/sec)). The levels of CSF $\alpha$-syn oligomers were significantly higher in the PD group (mean $\pm$ SEM = 19,791 $\pm$ 2,458, n = 32) than that in the age-matched control subjects (9,569 $\pm$ 1037, n = 28) (p < 0.0001, Mann-Whitney U test). Since measurement of CSF total $\alpha$-syn and measurement of CSF $\alpha$-syn oligomers from each subject were obtained in RLU/sec, these permit calculation of the ratio of $\alpha$-syn oligomers to the total $\alpha$-syn (oligomer/total ratio; %) in CSF for each patient. Interestingly, the ratio in CSF was significantly higher in the PD group (mean $\pm$ SEM = 12.92 $\pm$ 1.08, n = 32) compared with that in the control group (4.58 $\pm$ 0.52, n = 28) (p < 0.0001, Mann-Whitney U test) (Figure 1C). There was no significant difference in the ratio between male and female PD patients, as well as between patients with and without dementia (data not shown). Furthermore, the CSF levels of $\alpha$-syn oligomers did not have any correlation with subject's age (r = 0.06, p = 0.73), Hoehn-Yahr grade (r = -0.12, p = 0.51), or disease duration (r = 0.01, p = 0.95). Neither was there any correlation of the oligomers/total $\alpha$-syn ratio with subject's age (r = -0.17, p = 0.34), Hoehn-Yahr grade (r = -0.14, p = 0.44), or disease duration (r = -0.02, p = 0.89). Interestingly, the levels of $\alpha$-syn oligomers and the oligomers/total $\alpha$-syn ratio were even higher in patients with mild PD (Hoehn-Yahr grade 1 and 2, n = 8, p = 0.0046 for the oligomers levels, and p = 0.0002 for the ratio), and for the patients with early PD (within 24 months after the onset, n = 12, p = 0.0002 for oligomer levels, and p < 0.0001 for the ratio), compared with control group.

Figure 2 shows ROC curve for CSF $\alpha$-syn oligomers and the ratio (oligomer/total; %) of CSF $\alpha$-syn in discrimination of PD from controls. The ROC curve demonstrated that cutoff values of 9,950 RLU/sec for the CSF $\alpha$-syn oligomers and 6.165% for the oligomer/total most reliably distinguished PD from the control group. The cutoff level of 9,950 RLU/sec for the CSF $\alpha$-syn oligomers yielded a sensitivity of 75.0% (95% CI, 55.1 to 89.3%), and a specificity of 87.5% (95% CI, 71.0 to 96.5%) with an area under curve (AUC) of 0.859. Whereas the cutoff level of 6.165% for the oligomer/total ratio yielded a sensitivity of 89.3% (95% CI, 71.8 to 97.7%) and a specificity of 90.6% (95% CI, 75.0 to 98.0%) with an AUC of 0.948.

These results demonstrate that quantification of total and oligomeric forms of $\alpha$-syn in CSF has a strong value as a tool not only for the diagnosis of patients with PD, but the pre-symptomatic screening of high-risk individuals who are good candidates for neuroprotective treatment.

Table 1. Clinical details of patients with PD and the levels of total and oligomeric $\alpha$-synuclein in CSF

| Case | Gender | Age (y) | Disease dration (m) | H-Y grade | Bulbar sign | total $\alpha$-syn (ng/ml) | $\alpha$-syn oligomer (RLU/sec) | oligomer/total ratio (%) |
|---|---|---|---|---|---|---|---|---|
| 1 | M | 67 | 12 | 2 | - | 19.7 | 18,095 | 13.1 |

(continued)

| Case | Gender | Age (y) | Disease dration (m) | H-Y grade | Bulbar sign | total α-syn (ng/ml) | α-syn oligomer (RLU/sec) | oligomer/total ratio (%) |
|---|---|---|---|---|---|---|---|---|
| 2 | M | 60 | 60 | 3 | + | 15.0 | 13,315 | 14.4 |
| 3 | M | 77 | 60 | 2 | + | 30.9 | 81,535 | 30.8 |
| 4 | M | 83 | 12 | 3 | - | 19.4 | 8,863 | 6.6 |
| 5 | M | 80 | 6 | 3 | - | 19.0 | 30,742 | 23.6 |
| 6 | F | 59 | 36 | 3 | - | 12.5 | 11,794 | 16.7 |
| 7 | M | 67 | 24 | 3 | - | 23.6 | 18,411 | 10.3 |
| 8 | M | 70 | 36 | 4 | - | 22.8 | 24,921 | 14.7 |
| 9 | M | 64 | 36 | 2 | - | 17.9 | 8,494 | 7.1 |
| 10 | M | 65 | 24 | 3 | - | 20.0 | 12,156 | 8.7 |
| 11 | F | 71 | 6 | 3 | - | 23.1 | 11,953 | 6.9 |
| 12 | F | 43 | 48 | 3 | - | 22.5 | 23,233 | 13.9 |
| 13 | F | 70 | 120 | 4 | - | 24.9 | 20,624 | 10.7 |
| 14 | M | 74 | 24 | 3 | - | 19.4 | 18,578 | 13.8 |
| 15 | F | 76 | 9 | 3 | - | 18.9 | 14,875 | 11.5 |
| 16 | F | 51 | 18 | 2 | - | 14.9 | 17,897 | 19.6 |
| 17 | M | 77 | 180 | 4 | - | 20.4 | 10,483 | 7.2 |
| 18 | M | 66 | 72 | 3 | - | 27.5 | 26,038 | 11.7 |
| 19 | F | 79 | 20 | 2 | - | 21.8 | 8,606 | 5.4 |
| 20 | M | 56 | 60 | 4 | - | 23.2 | 28,945 | 16.7 |
| 21 | F | 57 | 72 | 3 | - | 18.8 | 18,867 | 14.7 |
| 22 | F | 59 | 228 | 3 | - | 19.0 | 24,257 | 18.6 |
| 23 | F | 69 | 36 | 1 | - | 17.8 | 11,621 | 9.8 |
| 24 | F | 72 | 24 | 3 | + | 25.4 | 11,406 | 5.8 |
| 25 | M | 56 | 16 | 1 | - | 25.1 | 23,548 | 12.1 |
| 26 | F | 66 | 24 | 3 | - | 17.3 | 29,272 | 25.7 |
| 27 | M | 53 | 60 | 4 | - | 16.8 | 14,359 | 13.1 |
| 28 | F | 75 | 15 | 3 | - | 31.2 | 44,073 | 16.4 |
| 29 | F | 71 | 72 | 4 | - | 23.4 | 11,188 | 6.4 |
| 30 | M | 75 | 96 | 3 | - | 22.9 | 11,792 | 6.9 |
| 31 | M | 76 | 6 | 2 | - | 16.1 | 15,510 | 15.2 |
| 32 | M | 70 | 70 | 4 | - | 20.2 | 7,873 | 5.5 |

y = years; m = months; M = male; F = female; H-Y grade = Hoehn-Yahr grade; RLU = relative luminescence unit; oligomer/total ratio = the ratio of α-synuclein oligomers to total α-synuclein in CSF.

## Example 2

A larger cross-sectional study examined CSF samples from another cohort of 121 subjects, including patients clinically diagnosed with PD (n=25, aged 43-83 [mean ± SD, 68.6 ±10.4] years), Alzheimer's Disease (AD) (n=35, aged 58-83 [mean ± SD, 74.1 ± 5.6] years) and progressive supranuclear palsy (PSP) (n=18, aged 51-78 [mean ± SD, 69.4 ±8.1] years), and control subjects (n=43, aged 35-88 [mean ± SD, 64.9 ±12.5] years). The patients with AD fulfilled DSMIV criteria for AD and NINCDS-ADRDA criteria for the clinical diagnosis of 'probable AD' (McKhann et al; Neurology 1984; 34 p939-944). Patients with PSP fulfilled the NINDS-SPSP diagnostic criteria for clinically definite or clinically probable PSP (Lityan et al; Mov. Disord 2003; 18 p467-486).

The CSF samples were analysed for $\alpha$-syn levels by ELISA as described above. As in the first cohort, significantly higher levels of CSF $\alpha$-syn oligomers were observed in PD cases (n=25) compared to PSP (n=18; $p < 0.05$, Dunn's multiple comparison test), AD (n=35; $p < 0.001$) and control subjects (n=43; $p < 0.05$) (Figure 3). This confirms the results for the first cohort, and also indicates that $\alpha$-syn in CSF is useful to distinguish PD from other neurological disorders.

**Claims**

1. A method of identifying whether or not an individual has Parkinson's disease (PD) and/or distinguishing between PD and another neurological disorder which is not PD, which method comprises measuring the amount of soluble $\alpha$-synuclein oligomers and the total amount of $\alpha$-synuclein in a cerebrospinal fluid (CSF) sample taken from the individual, calculating the ratio of:

$$\frac{\text{amount of } \alpha\text{-synuclein oligomers}}{\text{total amount of } \alpha\text{-synuclein}}$$

and thereby determining whether or not the individual has PD.

2. A method according to claim 1, wherein determining whether or not the individual has PD comprises determining whether or not the ratio is increased relative to the ratio in a sample taken from a non-PD individual.

3. A method according to claim 2, wherein the individual is determined as having PD when the ratio is increased by at least 3 fold relative to the ratio in sample taken from a non-PD individual.

4. A method according to claim 1, wherein determining whether or not the individual has PD comprises determining whether or not the ratio, when expressed as a percentage, is at least 6%.

5. A method according to any one of the preceding claims, wherein the individual is suspected of being at risk of developing PD or another neurological disorder which is not PD.

6. A method according to claim 5, wherein the individual has a familial history of PD or another neurological disorder which is not PD.

7. A method according to claim 5 or 6, wherein the individual (a) does not have or has not been diagnosed with any of the clinical symptoms associated with a diagnosis of PD, or (b) has or has been diagnosed with any one of the non-motor symptoms of PD, or (c) has or has been diagnosed with one or more symptoms associated with another neurological disorder which is not PD.

8. A method according to any one of the preceding claims, wherein the individual is categorised as Hoehn-Yahr grade 2 or lower.

**Patentansprüche**

1. Verfahren zur Identifizierung, ob ein Individuum Parkinsonsche Erkrankung (PD) hat oder nicht, und/oder zum Unterscheiden zwischen PD und einer anderen neurologischen Störung, welche nicht PD ist, wobei das Verfahren Messen der Menge an löslichen $\alpha$-Synuclein-Oligomeren und der Gesamtmenge an $\alpha$-Synuclein in einer Zerebro-spinalflüssigkeits (ZSF)-Probe, die dem Individuum entnommen worden war, Berechnen des Verhältnisses:

$$\frac{\text{Menge an } \alpha\text{-Synuclein-Oligomeren}}{\text{Gesamtmenge von } \alpha\text{-Synuclein}}$$

und dadurch Bestimmen, ob das Individuum PD hat oder nicht, umfasst.

**2.** Verfahren gemäß Anspruch 1, wobei Bestimmen, ob das Individuum PD hat oder nicht, Bestimmen, ob das Verhältnis relativ zu dem Verhältnis in einer Probe, die einem Nicht-PD-Individuum entnommen worden war, erhöht ist oder nicht, umfasst.

**3.** Verfahren gemäß Anspruch 2, wobei das Individuum als PD aufweisend bestimmt wird, wenn das Verhältnis relativ zu dem Verhältnis in einer Probe, die von einem Nicht-PD-Individuum entnommen worden war, wenigstens dreifach erhöht ist.

**4.** Verfahren gemäß Anspruch 1, wobei Bestimmen, ob das Individuum PD hat oder nicht, Bestimmen, ob das Verhältnis, wenn es als Prozentwert ausgedrückt wird, wenigstens 6% ist oder nicht.

**5.** Verfahren gemäß einem der vorangehenden Ansprüche, wobei vermutet wird, dass das Individuum ein Risiko für die Entwicklung von PD oder einer anderen neurologischen Störung, die nicht PD ist, hat.

**6.** Verfahren gemäß Anspruch 5, wobei das Individuum eine familiäre Vorgeschichte von PD oder einer anderen neurologischen Störung, die nicht PD ist, hat.

**7.** Verfahren gemäß Anspruch 5 oder 6, wobei das Individuum (a) keins der klinischen Symptome hat, die mit der Diagnose von PD assoziiert sind, oder keins der klinischen Symptome, die mit der Diagnose von PD assoziiert sind, bei ihm diagnostiziert wurde, oder (b) eins der Nicht-Motor-Symptome von PD hat oder eins der Nicht-Motor-Symptome von PD bei ihm diagnostiziert wurde, oder (c) ein oder mehrere Symptome, die mit einer anderen neurologischen Störung, die nicht PD ist, assoziiert sind, hat oder ein oder mehrere Symptome, die mit einer anderen neurologischen Störung, die nicht PD ist, bei ihm diagnostiziert wurde(n).

**8.** Verfahren gemäß einem der vorangehenden Ansprüche, wobei das Individuum als Hoehn-Yahr-Grad 2 oder niedriger kategorisiert wird.

**Revendications**

**1.** Procédé permettant d'établir si, oui ou non, un individu est atteint de la maladie de Parkinson (MP) et/ou de distinguer entre la MP et un autre trouble neurologique qui n'est pas la MP, lequel procédé comporte

- le fait de mesurer la quantité d'oligomères solubles d'α-synucléine et la quantité totale d'α-synucléine dans un échantillon de liquide cérébrospinal (LCS) prélevé chez l'individu ;
- le fait de calculer le rapport

$$\frac{\text{quantité d'oligomères d'}\alpha\text{-synucléine}}{\text{quantité totale d'}\alpha\text{-synucléine}}$$

- et le fait de déterminer par là si, oui ou non, l'individu est atteint de la MP.

**2.** Procédé conforme à la revendication 1, dans lequel établir si, oui ou non, l'individu est atteint de la MP comporte le fait de déterminer si, oui ou non, le rapport est plus grand que le rapport trouvé dans un échantillon prélevé chez un individu non atteint de la MP.

**3.** Procédé conforme à la revendication 2, dans lequel il est établi que l'individu est atteint de la MP si le rapport vaut au moins le triple du rapport trouvé dans un échantillon prélevé chez un individu non atteint de la MP.

**4.** Procédé conforme à la revendication 1, dans lequel établir si, oui ou non, l'individu est atteint de la MP comporte le fait de déterminer si, oui ou non, le rapport, exprimé en pourcentage, vaut au moins 6 %.

**5.** Procédé conforme à l'une des revendications précédentes, dans lequel l'individu est suspecté de risquer de développer la MP ou un autre trouble neurologique qui n'est pas la MP.

**6.** Procédé conforme à la revendication 5, dans lequel l'individu présente des antécédents familiaux de MP ou d'un autre trouble neurologique qui n'est pas la MP.

**7.** Procédé conforme à la revendication 5 ou 6, dans lequel l'individu

a) ne présente, ou l'on n'a diagnostiqué chez lui, aucun des symptômes cliniques associés à un diagnostic de MP,
b) ou présente, ou l'on a diagnostiqué chez lui, l'un des symptômes non-moteurs de MP,
c) ou présente, ou l'on a diagnostiqué chez lui, un ou plusieurs des symptômes associés à un autre trouble neurologique qui n'est pas la MP.

**8.** Procédé conforme à l'une des revendications précédentes, dans lequel l'individu est classé, sur l'échelle de Hoehn et Yahr, au stade 2 ou à un stade inférieur.

FIGURE 1

EP 2 478 375 B1

FIGURE 2

EP 2 478 375 B1

FIGURE 3

EP 2 478 375 B1

**EP 2 478 375 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **EL-AGNAF et al.** *FASEB Journal,* 2006, vol. 20 (3), 419-425
- **MCKHANN et al.** *Neurology,* 1984, vol. 34, 939-944
- **LITYAN et al.** *Mov. Disord,* 2003, vol. 18, 467-486